Europäisches Patentamt

European Patent Office

Office européen des brevets

(11) Veröffentlichungsnummer: **0 300 114 B1**

(12) **EUROPÄISCHE PATENTSCHRIFT**

(45) Veröffentlichungstag der Patentschrift: **10.06.92**

(21) Anmeldenummer: **88100702.5**

(22) Anmeldetag: **20.01.88**

(51) Int. Cl.⁵: **A61M 25/00, A61M 5/14**

(54) **Kathetervorrichtung.**

(30) Priorität: **27.06.87 DE 3721299**

(43) Veröffentlichungstag der Anmeldung:
**25.01.89 Patentblatt 89/04**

(45) Bekanntmachung des Hinweises auf die
Patenterteilung:
**10.06.92 Patentblatt 92/24**

(84) Benannte Vertragsstaaten:
**AT BE CH DE ES FR GB GR IT LI NL SE**

(56) Entgegenhaltungen:
**DE-A- 3 201 954          DE-C- 395 389**
**DE-C- 3 540 946          GB-A- 2 044 109**
**US-A- 1 920 006          US-A- 3 799 172**
**US-A- 3 841 308**

(73) Patentinhaber: **Haindl, Hans, Dr.**
**Hauptstrasse 39**
**W-3015 Wennigsen(DE)**

(72) Erfinder: **Haindl, Hans, Dr.**
**Hauptstrasse 39**
**W-3015 Wennigsen(DE)**
Erfinder: **Fuchs, Jürgen**
**Wolfhager Strasse 45**
**W-3501 Emstal-Sand(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Kathetervorrichtung mit Verweilkatheter bzw. Kurzkatheter.

Bekannt sind Kurzkatheter, deren Kanülenrohr über eine Stahlkanüle in die Vene eines Armes eingeschoben wird. Danach wird das am rückwärtigen Ende des Kanülenrohres befindliche Anschlußstück an dem Arm fixiert wird. In der Nähe des Anschlußstücks ist ein Zuspritzport vorgesehen, durch den dem Patienten flüssige Medikamente verabreicht werden können. Die Kathetervorrichtung weist einen Mandrin auf, der durch das Anschlußstück hindurch in das Kanülenrohr eingeschoben wird und dieses versperrt, wenn keine Injektion von Medikamenten bzw. keine Infusion erfolgt. Der Mandrin verhindert, daß dann, wenn keine Injektion durch den Katheter erfolgt, Blutbestandteile in den Katheter eindringen und zur Thrombenbildung führen. Ferner sorgt der Mandrin dafür, daß keine Kontaminationen in den Katheter und von dort in den Körper des Patienten eindringen können. Bei den bekannten Kurzkathetern besteht der Nachteil, daß vor jedem Injektionsvorgang der Mandrin entfernt werden muß, um die Verbindung vom Zuspritzport zum Kanülenrohr freizugeben und den Verschluß des Kanülenrohres aufzuheben. Nach Beendigung der Injektion muß ein steriler neuer Mandrin eingeschoben werden. In der Praxis wird jedoch häufig derselbe Mandrin eingeschoben, der vor der Injektion aus dem Verweilkatheter herausgezogen worden war. Solche Manipulationen sind mit einem hohen Kontaminationsrisiko behaftet. Außerdem ist das Hantieren mit dem Mandrin bzw. den Mandrins für den Anwender sehr umständlich.

Aus DE-C-395 389 ist eine Saugmannsche Kanüle zum Einblasen von Luft in den menschlichen Brustkorb bekannt, die ein starres Kanülenrohr aufweist, in das ein Mandrin eingeführt werden kann, der am patientenseitigen vorderen Ende eine Verdickung aufweist. Während des Einführens der Kanüle in den Körper des Patienten verschließt die Verdickung das vordere Kanülenende. Nach Beendigung des Einführens wird der Mandrin vorgeschoben, so daß die Verdickung aus dem Kanülenrohr heraus vorsteht und die Luft frei durch das Rohr hindurch in die Brusthöhle eintreten kann. Hierbei handelt es sich nicht um einen Venenverweilkatheter, der ein flexibles Kanülenrohr aufweist und über längere Zeit im Körper des Patienten verbleiben kann, um gelegentlich Medikamente zuspritzen zu können. Das Einblasen von Luft in den Brustkorb erfordert stets das manuelle Öffnen des Ventils zum Herausschieben der Verdickung aus dem Kanülenrohr.

Bekannt ist ferner ein Katheter, bei dem der Katheterschlauch am patientenseitigen vorderen Ende ein Rückschlagventil aufweist (DE-C-3 540 949). Das Rückschlagventil ist am Katheterschlauch ortsfest vorgesehen. Es öffnet, wenn im Katheterschlauch ein Überdruck auftritt. Dieses kann bei eingeschobenem Mandrin jedoch nicht benutzt werden.

Der Erfindung liegt die Aufgabe zugrunde, eine Kathetervorrichtung der im Oberbegriff des Patentanspruchs 1 angegebenen Art zu schaffen, die zum Zuspritzen von Medikamenten einfach zu bedienen ist und bei der das Kontaminationsrisiko und das Risiko der Thrombosierung des Kanülenrohres vermieden werden.

Die Lösung dieser Aufgabe erfolgt erfindungsgemäß mit den in einem der Patentansprüche 1 bis 3 angegebenen Merkmalen.

Nach der Erfindung wirken Mandrin und Kanülenrohr am patientenseitigen Ende als Ventil zusammen, während ein Flüssigkeitskanal sich bei im Katheter befindlichem Mandrin vom Zuspritzport bis zum Ventil erstreckt. Dadurch ist es möglich, auch bei im Katheter enthaltenem Mandrin Medikamente durch den Zuspritzport zu injizieren. Der Mandrin braucht also zum Injizieren von Medikamenten nicht aus dem Katheter herausgezogen zu werden, sondern er kann ständig im Katheter verbleiben. Andererseits ist der Mandrin ein selbständiges Teil, das erst nach der Verlegung des Katheters in diesen eingeführt wird, so daß der Katheter auf einer metallischen Punktionskanüle in den Körper des Patienten eingeführt werden kann. Nach dem Einführen des Katheters wird die Punktionskanüle aus dem Katheter herausgezogen, und der Mandrin wird in den Katheter eingeschoben. Das Katheterlumen wird also nicht durch fest angebrachte Teile blockiert.

Die Erfindung eignet sich insbesondere für Kurzkatheter, die als Verweilkatheter benutzt werden und bei denen das flexible Kanülenrohr eine Länge von etwa 4 bis 5 cm hat.

Die Erfindung bietet den Vorteil, daß der Mandrin auch für mehrere Injektionen nur ein einziges Mal in den Verweilkatheter eingeführt wird und dort verbleiben kann. Wenn keine Injektion erfolgt, verhindert das gesperrte Ventil das Eindringen von Blutbestandteilen in das Kanülenrohr, so daß die Gefahr der Thrombosierung des Kanülenrohres vermieden wird. Ferner wirkt der im Kanülenrohr verbleibende Mandrin als Kontaminationsschutz, weil sein rückwärtiges Anschlußstück den Kanülenkanal gegen die Umgebung verschließt und weil der Mandrin nach seiner Einführung in den Verweilkatheter nicht mehr entfernt werden muß.

Bei den in den Patentansprüchen 1 und 2 angegebenen Lösungen wird der Flüssigkeitskanal zwischen dem Mandrin und dem Kanülenrohr gebildet, wodurch das Medikament mit geringem Strömungswiderstand zum Ventil gelangt. Das Ventil ist ein bei geringem Überdruck öffnendes Rückschlagventil, durch das das Medikament in den Körper des Patienten hinein austritt. Dieses Rückschlagventil wird ebenfalls von Madrin und Katheterrohr am vorderen Ende des Kanülenrohres gebildet. Der Mandrin sollte mindestens bis zum vorderen Ende des Katheterrohres reichen; er kann auch geringfügig aus diesem heraus vorstehen.

Bei der im Patentanspruch 3 angegebenen Lösung verläuft der Flüssigkeitskanal im Innern des hohlen Mandrins, und das Ventil wird von dem vorderen Ende des Mandrins gebildet. Hierbei kann der Zuspritzport in der üblichen Weise seitlich am Anschlußstück des Katheters vorgesehen sein; es besteht aber auch die Möglichkeit, den Zuspritzport am rückwärtigen Ende des Mandrins vorzusehen, so daß durch den Mandrin hindurch, über dessen gesamte Länge, injiziert oder infundiert wird. In diesem Fall ist ein Zuspritzport an der Verweilkanüle überhaupt nicht erforderlich.

Nach der Erfindung dichtet der Mandrin den Flüssigkeitskanal mit einem Rückschlagventil am patientenseitigen Ende ab, so daß keine Blutbestandteile in das Kanülenrohr einfließen können. Bei dem Rückschlagventil ändert sich die Form des Kanülenrohrendes oder des Mandrinendes unter dem Druck des injizierten Medikamentes in einer Weise, daß das Medikament austreten kann. Dabei soll ein scharfer Medikamentenstrahl vermieden werden.

Für die Funktion des Rückschlagventils gibt es zwei Möglichkeiten: Einmal kann das Rückschlagventil durch radiale Deformierung von Katheterrohr oder Mandrin öffnen und zum anderen kann es durch axiale Streckung des Mandrins unter dem Injektionsdruck öffnen.

Im folgenden werden unter Bezugnahme auf die Zeichnungen Ausführungsbeispiele der Erfindung näher erläutert.

Es zeigen:

Fig. 1 die Einzelteile eines kompletten Kathetersets,

Fig. 2 einen Schnitt durch den Verweilkatheter entlang der Linie II-II von Fig. 1,

Fig. 3 einen Querschnitt entlang der Linie III-III von Fig. 2 bei einer ersten Ausführungsform der Kathetervorrichtung,

Fig. 4 einen Längsschnitt durch eine zweite Ausführungsform des Rückschlagventils,

Fig. 5 einen Längsschnitt durch eine dritte Ausführungsform des Rückschlagventils,

Fig. 6 einen Querschnitt entlang der Linie VI-VI von Fig. 5,

Fig. 7 einen Längsschnitt eines Ausführungsbeispiels mit wendelförmigem Mandrin,

Fig. 8 einen Längsschnitt durch eine Ausführungsform mit hohlem Mandrin,

Fig. 9 einen Längsschnitt einer Ausführungsform mit elastisch bewegbarem Kopfstück des Mandrins,

Fig. 10 einen Querschnitt entlang der Linie X-X in Fig. 9, und

Fig. 11 eine Ausführungsform des Mandrins mit integriertem Federelement,

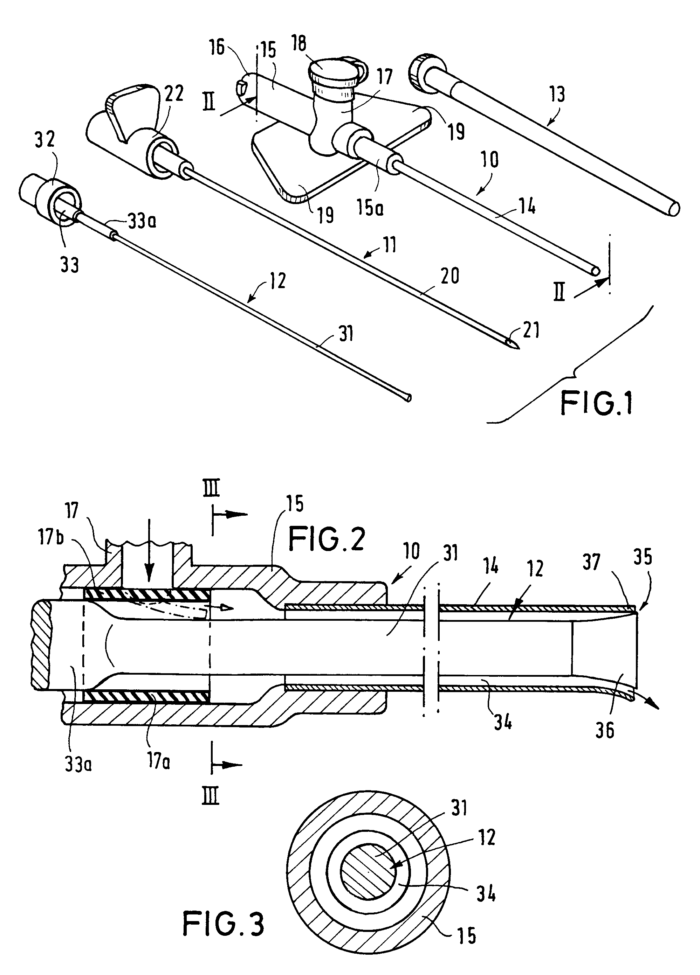

In Fig. 1 ist ein Katheterset dargestellt, der aus dem Verweilkatheter 10, der metallischen Punktionskanüle 11, dem Mandrin 12 und einer Schutzkappe 13 besteht.

Der Verweilkatheter 10 weist ein gerades langgestrecktes flexibles Kanülenrohr 14 aus Kunststoff auf, das eine Länge von etwa 4 bis 5 cm hat. Am rückwärtigen Ende des Kanülenrohres 14 befindet sich das Anschlußstück 15 mit einem Anschlußstutzen 16 und einem seitlichen Zuspritzport 17. Der Kanal des Anschlußstutzens 16 liegt in axialer Ausrichtung mit demjenigen des Kanülenrohres 14. Der Zuspritzport 17 weist eine abnehmbare Verschlußkappe 18 auf, die entfernt werden kann, um an den Zuspritzport 17 eine Spritze, oder eine andere Flüssigkeitsquelle anschließen zu können. Am Anschlußstück 15 befinden sich ferner seitlich abstehende Flügel 19, mit denen der Verweilkatheter 10 auf der Haut des Patienten fixiert werden kann, indem beispielsweise die Flügel 19 mit Pflaster an der Haut festgeklebt werden, während das Kanülenrohr 14 flach durch die Haut hindurch in eine Vene hineinragt.

Die Punktionskanüle 11 weist ein metallisches Rohr 20 auf, das am vorderen Ende mit einem Anschliff 21 zum Durchdringen der Haut versehen ist. Am rückwärtigen Kanülenende befindet sich ein Anschlußstück 22, das mit dem Anschlußstutzen 16 zusammenwirken und mit diesem verriegelt werden kann. Der Anschlußstutzen 16 und das Anschlußstück 22 bilden eine Luer-Lok-Verbindung, bei der beide Teile durch Drehen miteinander verschraubt und dabei abdichtend verbunden werden. Wenn das Anschlußstück 22 auf dem Anschlußstutzen 16 sitzt, ragt das Rohr 20 durch den Anschlufstutzen 16 und das Kanülenrohr 14 hindurch, wobei die Rohrspitze mit dem Anschliff 21 aus dem Kanülenrohr 14 herausragt. In diesem Zustand kann die Punktion durchgeführt werden, wobei das Rohr 20, das von dem Kanülenrohr 14 eng umschlossen ist, in die Vene eingeschoben wird. Nach dem Punktieren der Vene wird die Punktionskanüle 11 aus dem Verweilkatheter 10 herausgezogen, und dieser wird auf der Haut des Patienten fixiert. Danach wird der Mandrin 12 durch den Anschlußstutzen 16 hindurch in den Verweilkatheter 10 eingeschoben. Der Mandrin 12 weist einen langgestreckten Stab 31 auf, der an seinem rückwärtigen Ende ein Anschlußstück 32 trägt, das mit dem Anschlußstutzen 16 abdichtend verriegelt werden kann. Wenn das Anschlußstück 32 auf dem Anschlußstutzen 16 sitzt, reicht das vordere Ende des Stabes 31 bis zum vorderen Ende des Kanülenrohres 14. Zwischen dem Anschlußstück 32 und dem Stab 31 befindet sich ein Versteifungsbereich 33a, der mit dem Kern 33 des Anschlußstücks 32 verbunden ist. Das Anschlußstück 32 ist als weiblicher Luer-Konus ausgebildet.

Im verpackten Zustand des Kathetersets ist das Rohr 20 der Punktionskanüle 11 in den Verweilkatheter 10 eingeschoben, und die Schutzkappe 13 ist über das Kanülenrohr 14 geschoben, so daß sie den Anschliff 21 bedeckt. Das rückwärtige Ende der Schutzkappe 13 wird auf einem Ansatz 15a des Anschlußstücks 15 klemmend festgehalten. Sämtliche Teile des Kathetersets sind in einer keimdichten Packung steril verpackt.

Fign. 2 und 3 zeigen ein erstes Ausführungsbeispiel, bei dem der Stab 31 des Mandrins 12 kreisförmigen Querschnitt hat und der Durchmesser des Mandrins so klein ist, daß zwischen dem Innenlumen des Kanülenrohres 14 und dem Mandrin 12 ein ringförmiger Flüssigkeitskanal 34 verläuft. Dieser Flüssigkeitskanal 34 verbindet den Zuspritzport 17 mit dem am vorderen Katheterende vorgesehenen Rückschlagventil 35. Das Rückschlagventil 35 besteht bei diesem Ausführungsbeispiel aus einem sich im Querschnitt erweiternden Kopfstück 36 am vorderen Ende des Stabes 31 und aus dem radial aufweitbaren Endabschnitt 37 des Kanülenrohres 14. Wenn keine Injektion erfolgt, umschließt der Endabschnitt 37 das Kopfstück 36. Während einer Injektion öffnet dagegen das Rückschlagventil, indem der Endabschnitt 37 aufgeweitet wird und vom Kopfstück 36 abhebt. Auf diese Weise entsteht ein ringförmiger Auslaß. In Fig. 2 ist in der oberen Hälfte die Schließposition und in der unteren Hälfte die Öffnungsposition des Rückschlagventils 35 dargestellt.

Der Versteifungsbereich 33a des Mandrins 12 hat rechteckigen Querschnitt, der den Flüssigkeitskanal 34 im Innern des Anschlußstückes 15 nicht ausfüllt, so daß der Versteifungsbereich 33a seitlich umströmt werden kann. Der Versteifungsbereich 33a ermöglicht ferner das Einströmen von Flüssigkeit aus dem Zuspritzport 17 in den Flüssigkeitskanal 34. Im Anschlußstück 15 befindet sich in demjenigen Bereich, in den der Zuspritzport 17 mündet, ein Schlauchstück 17a, das an seinem rückwärtigen Ende 17b an der Wand des Anschlußstücks 15 festgeklebt ist, während sein Umfang im übrigen lose an der Innenwand des Anschlußstücks 15 anliegt. Das Schlauchstück 17a bildet ein Ventil, das den Zuspritzport 17 vom Innern des Anschlußstücks 15 trennt und durch Flüssigkeitsdruck im Zuspritzport 17 geöffnet wird.

Wenn keine Flüssigkeit durch den Zuspritzport 17 injiziert wird, ist das Rückschlagventil 35 geschlossen, so daß keine Blutbestandteile in das Kanülenrohr 14 eindringen können. Das Rückschlagventil 35 wird nur durch den Druck im Innern des Kanülenrohres 14 geöffnet, wenn dieser Druck größer ist als der Venendruck. Der Mandrin 12 bleibt auch während der Nicht-Injektionszeiten in dem Verweilkatheter.

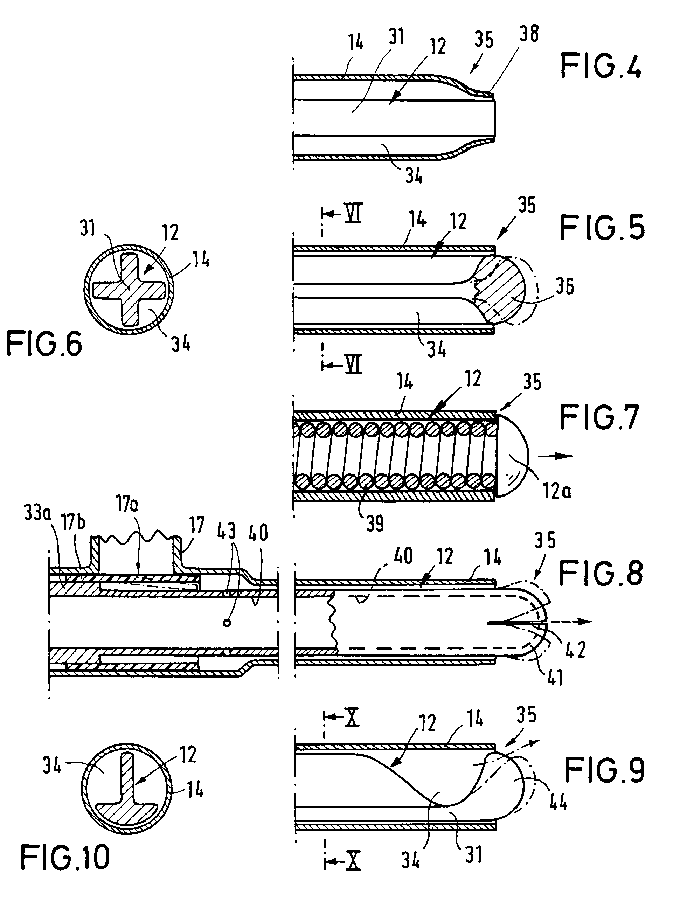

Bei dem Ausführungsbeispiel von Fig. 4 weist das Kanülenrohr 14 am vorderen Ende einen Verengungsbereich 38 auf, in welchem sich der Innendurchmesser des Kanülenrohres bis auf den Außendurchmesser des Mandrins 12 reduziert. Auf diese Weise entsteht am vorderen Ende eine Versperrung des Flüssigkeitskanals 34. Der Verengungsbereich 38 ist, z.B. durch Verringerung der Materialstärke, elastisch aufweitbar, so daß die Flüssigkeitssperre durch den Injektionsdruck überwunden wird.

Bei dem Ausführungsbeispiel der Fign. 5 und 6 ist der Schaft 31 so ausgebildet, daß sein Umfang zwar an der Innenwand des Kanülenrohres 14 anliegt, den Schaftquerschnitt des Kanülenrohres aber nicht ausfüllt. Dies wird im vorliegenden Fall durch eine kreuzförmige Gestalt des Schaftquerschnitts erreicht. Am vorderen Ende des Mandrins 12 befindet sich ein Kopfstück 36, das den gesamten Querschnitt des Kanülenrohres 14 ausfüllt und geringfügig aus dem Kanülenrohr heraus vorsteht. Beim Zuspritzen des Medikaments wandert das Kopfstück unter dem Injektionsdruck nach vorne, so daß es das Innere des Kanülenrohres 14 verläßt und das Medikament aus dem Flüssigkeitskanal 34 austreten kann. Die Längung des Mandrins 12 zum Öffnen des Rückschlagventils 35 beträgt etwa 0,5 mm.

Bei dem Ausführungsbeispiel von Fig. 7 besteht der Mandrin 12 aus einer schraubenförmigen Wendel 39 aus zahlreichen Windungen aus Metall oder Kunststoff, die an ihrem patientenseitigen Ende durch Zuschweißen oder Schmelzen mit einem Kopfstück 12a verschlossen ist, das am vorderen Ende des Kanülenrohrs 14 abdichtend anliegt. Der Flüssigkeitskanal wird innerhalb der Wendel 39 gebildet. Durch den Injektionsdruck wird die Wendel 39 gedehnt, so daß sich der Mandrin 12 verlängert und das Kopfstück 12a von dem Ende des Kanülenrohrs 14 abhebt. Dabei heben die Windungen der Wendel 39, die zuvor eng aneinandergelegen haben, voneinander ab.

Fig. 8 zeigt eine Ausführungsform, bei der der Mandrin 12 hohl ausgebildet ist und einen längslaufenden Flüssigkeitskanal 40 enthält. Der Außendurchmesser des Mandrins 12 entspricht im wesentlichen dem Innendurchmesser des Kanülenrohres 14, und das vordere Ende 41 des Mandrins 12 ragt aus dem Kanülenrohr 14 heraus. Dieses vordere Ende 41 bildet das Rückschlagventil 35. Es ist mit einem oder mehreren Querschlitzen 42 versehen, der/die das vordere Ende 41 in zwei Hälften oder mehrere Segmente unterteilt, die nach Art eines Fischmaul-Ventils bei einem Innendruck radial ausweichen und sich nach entgegengesetzten Richtungen öffnen. Der Flüssigkeitskanal 40 steht mit dem Zuspritzport 17 über radiale Löcher 43 in der Mandrinwand in Verbindung. Der verdickte Versteifungsbereich 33a hat bei diesem Ausführungsbeispiel runden Querschnitt, und er liegt abdichtend an dem rückwärtigen Ende 17b des Schlauchstücks 17a an, während sich der vordere Bereich des Schlauchstücks 17a einwärts verformen kann.

In Fign. 9 und 10 ist eine Ausführungsform dargestellt, bei der der Mandrin 12 im wesentlichen T-förmigen Querschnitt hat, so daß im Innern des Kanülenrohres 14 Raum für den Flüssigkeitskanal 34 zur Verfügung steht. Das Rückschlagventil 35 besteht aus einem elastischen Kopfstück 44, das aus dem Ende des Kanülenrohres 14 herausragt und dieses Ende im Normalzustand verschließt.

Durch einen Injektionsdruck im Flüssigkeitskanal 34 weicht das Kopfstück 44, das nur auf einem Teilbereich seines Umfangs mit dem Stab 31 verbunden ist, aus, so wie dies in Fig. 9 strichpunktiert dargestellt ist. Durch das elastische radiale Ausweichen des Kopfstücks 44 wird ein Durchlaß am Ende des Kanülenrohres 14 geöffnet.

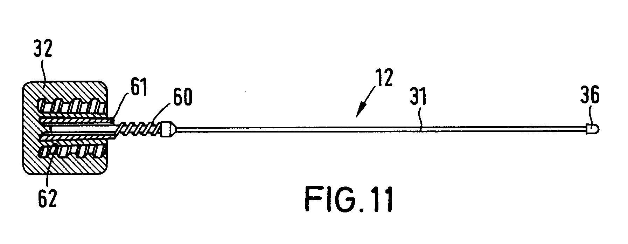

Der Mandrin 12 nach Fig. 11 weist einen langgestreckten Mandrinstab 31 mit Kopfstück 36 auf. Am rückwärtigen Ende des Mandrinstabes 36 befindet sich ein Federelement 60, das in Längsrichtung des Mandrinstabs federt und bei dem vorliegenden Ausführungsbeispiel aus einer Kunststoffoder Stahlspirale besteht. Das federnde Element 60 ist mit einer Hülse 61 verbunden, die in einer Muffe 62 des Anschlußstücks 32 befestigt ist. Das Anschlußstück 32 besteht aus einer Gewindekappe mit Innengewinde, die auf den Anschlußstutzen 16 des Verweilkatheters 10 (Fig. 1) aufgeschraubt wird. Die Gewindemuffe, die den Anschlußstutzen 16 übergreift, wirkt auch als Kontaminationsschutz zur Verhinderung des Eindringens von Verunreinigungen in das Innere des Anschlußstücks 15. Ein Flüssigkeitsdruck im Katheterrohr 14 drückt das Kopfstück 36 nach außen, wodurch das federnde Element 60 gedehnt wird und am forderen Ende des Katheterrohrs 14 eine Austrittsöffnung entsteht.

**Patentansprüche**

1. Kathetervorrichtung mit

einem Verweilkatheter (10), der ein Kanülenrohr (14) mit einem am rückwärtigen Ende vorgesehenen Anschlußstück (15) und einem seitlich einmündenden Zuspritzport (17) aufweist,

und einem durch das Anschlußstück (15) in den Verweilkatheter (10) einführbaren Mandrin (12), der sich im eingeführten Zustand bis zum patientenseitigen vorderen Kanülenrohrende erstreckt und dabei das vordere Ende des Kanülenrohres gegen das Einfließen von Blut versperrt, wobei zwischen Mandrin (12) und Kanülenrohr (14) ein längslaufender Flüssigkeitskanal (34) vorhanden ist und der Mandrin (12) zusammen mit dem Kanülenrohr (14) am vorderen Ende des Flüssigkeitskanals (34) ein Ventil bildet,

**dadurch gekennzeichnet,**

daß das Ventil ein durch den Überdruck im Flüssigkeitskanal (34) öffnendes Rückschlagventil (35) ist, das aus einem den Flüssigkeitskanal (34) versperrenden Kopfstück (36) am vorderen Ende des Mandrins (12) besteht, und daß der Mandrin in Längsrichtung derart dehnbar ist, daß das Kopfstück (36) sich bei Überdruck im Flüssigkeitskanal (34) von dem Kanülenrohr (14) entfernt.

2. Kathetervorrichtung mit

einem Verweilkatheter (10), der ein Kanülenrohr (14) mit einem am rückwärtigen Ende vorgesehenen Anschlußstück (15) und einem seitlich einmündenden Zuspritzport (17) aufweist,

und einem durch das Anschlußstück (15) in den Verweilkatheter (10) einführbaren Mandrin (12), der sich im eingeführten Zustand bis zum patientenseitigen vorderen Kanülenrohrende erstreckt und dabei das vordere Ende des Kanülenrohres gegen das Einfließen von Blut versperrt, wobei zwischen Mandrin (12) und Kanülenrohr (14) ein längslaufender Flüssigkeitskanal (34) vorhanden ist und der Mandrin (12) zusammen mit dem Kanülenrohr (14) am vorderen Ende des Flüssigkeitskanals (34) ein Ventil bildet,

**dadurch gekennzeichnet,**

daß das Rückschlagventil (35) aus einem elastisch aufweitbaren Verengungsbereich (38) am vorderen Ende des Kanülenrohres (14) besteht, welcher das vordere Ende des Mandrins (12) umschließt.

3. Kathetervorrichtung mit

einem Verweilkatheter (10), der ein Kanülenrohr (14) mit einem am rückwärtigen Ende vorgesehenen Anschlußstück (15) und einem seitlich einmündenden Zuspritzport (17) aufweist,

und einem durch das Anschlußstück (15) in den Verweilkatheter (10) einführbaren Mandrin (12), der sich im eingeführten Zustand bis zum patientenseitigen vorderen Kanülenrohrende erstreckt und dabei das vordere Ende des Kanülenrohres gegen das Einfließen von Blut versperrt, wobei ein längslaufender Flüssigkeitskanal vorhanden ist und der Mandrin (12) am vorderen Ende des Flüssigkeitskanals (34) ein Ventil bildet,

**dadurch gekennzeichnet,**

daß der Mandrin (12) hohl ist und den längslaufenden Flüssigkeitskanal (40) enthält und daß das Ventil am vorderen Ende des Mandrins (12) als durch den Überdruck im Flüssigkeitskanal (40) öffnendes geschlitztes Rückschlagventil (35) mit radial ausweichenden Segmenten ausgebildet ist.

4. Kathetervorrichtung nach Anspruch 2, dadurch gekennzeichnet, daß das Rückschlagventil (35) aus einem im Querschnitt vergrößerten, den Flüssigkeitskanal (34) versperrenden Kopfstück (36) am vorderen Ende des Mandrins (12) und einem das Kopfstück (36) umschließenden, elastisch aufweitbaren Endabschnitt (37) am vorderen Ende des Kanülenrohres (14) besteht.

5. Kathetervorrichtung nach einem der Ansprüche 1 oder 2, dadurch gekennzeichnet, daß der Mandrin (12) im Bereich des Flüssigkeitskanals (34) runden Querschnitt hat, dessen Durchmesser zur Bildung des Flüssigkeitskanals (34) kleiner ist als der Innendurchmesser des Kanülenrohres (14).

6. Kathetervorrichtung nach einem der Ansprüche 1 oder 2, dadurch gekennzeichnet, daß der Flüssigkeitskanal (34) aus längslaufenden Rillen des Mandrins (12) besteht.

7. Kathetervorrichtung nach Anspruch 1, dadurch gekennzeichnet, daß der Mandrin (12) eine Wendel (39) aufweist, deren Windungen lose gegeneinanderliegen und einen wendelförmigen Flüssigkeitskanal bilden, derart, daß bei einem Überdruck im Flüssigkeitskanal die Wendel unter Querschnittsvergrößerung des Flüssigkeitskanals axial gestreckt wird.

8. Kathetervorrichtung nach Anspruch 1, dadurch gekennzeichnet, daß der Mandrin (12) über ein axial federndes elastisches Element (60) mit dem Anschlußstück (15) derart verbunden ist, daß ein Kopfstück (36) des Rückschlagventils in die Schließstellung vorgespannt ist.

**Claims**

1. Catheter means comprising
   - a dwell-in catheter (10) having a cannula tube (14) with a connector (15) provided at its rear end and a injection port (17) opening laterally thereinto, and
   - a mandrin (12) insertable through the connector (15) into the dwell-in catheter (10) and extending in the introduced condition to the front cannula tube end directed to the patient, thereby blocking the front end of the cannula tube against blood inflow, a longitudinal fluid channel (34) being provided between mandrin (12) and cannula tube (14), the mandrin (12), together with the cannula tube (14), forming a valve at the front end of the fluid channel (34),

   **characterized in**

   that said valve is a nonreturn valve (35) to be opened by overpressure in the fluid channel (34) and comprising a headpiece (36) provided at the front end of the mandrin (12) and blocking the fluid channel (34), and that the mandrin is longitudinally extendable such, that the headpiece (36) moves away from the cannula tube (14) when overpressure prevails in the fluid channel (34).

2. Catheter means comprising
   - a dwell-in catheter (10) having a cannula tube (14) with a connector (15) provided at its rear end and a injection port (17) opening laterally thereinto, and
   - a mandrin (12) insertable through the connector (15) into the dwell-in catheter (10) and extending in the introduced condition to the front cannula tube end directed to the patient, thereby blocking the front end of the cannula tube against blood inflow, a longitudinal fluid channel (34) being provided between mandrin (12) and cannula tube (14), the mandrin (12), together with the cannula tube (14), forming a valve at the front end of the fluid channel (34),

   **characterized in**

   that the nonreturn valve (35) consists of an elastically expandable constricted portion (38) at the front end of the cannula tube (14), which encloses the front end of the mandrin (12).

3. Catheter means comprising
   - a dwell-in catheter (10) having a cannula tube (14) with a connector (15) provided at its rear end and a injection port (17) opening laterally thereinto, and
   - a mandrin (12) insertable through the connector (15) into the dwell-in catheter (10) and extending in the introduced condition to the front cannula tube end directed to the patient, thereby blocking the front end of the cannula tube against blood inflow, a longitudinal fluid channel (34) being provided between mandrin (12) and cannula tube (14), the mandrin (12), together with the cannula tube (14), forming a valve at the front end of the fluid channel (34),

**characterized in**

that the mandrin (12) is hollow and includes the longitudinally extending fluid channel (40) and that the valve at the front end of the mandrin (12) is designed as a slotted nonreturn valve (35) to be opened by over-pressure in the fluid channel (34) and having radially evading segments.

**4.** Catheter means as defined in claim 2, characterized in that the nonreturn valve (35) consists of a cross-sectionally enlarged headpiece (36) blocking the fluid channel (34) and being provided at the front end of the mandrin (12), and of a flexibly expandable end section (37) embracing the headpiece (36) at the front end of the cannula tube (14).

**5.** Catheter means as defined in one of claims 1 or 2, characterized in that, within the range of the fluid channel (34), the mandrin (12) has a round cross section, the diameter of which used for the formation of the fluid channel (34) is inferior to the inner diameter of the cannula tube (14).

**6.** Catheter means as defined in one of claims 1 or 2, characterized in that the fluid channel (34) consists of longitudinal grooves of the mandrin (12).

**7.** Catheter means as defined in claim 1, characterized in that the mandrin (12) contains a coil (39), the windings of which loosely rest against each other and form a helical fluid channel such that, in case of an overpressure in the fluid channel, the coil is axially stretched with an increase in cross section of the fluid channel.

**8.** Catheter means as defined in claim 1, characterized in that the mandrin (12) is connected with the connector (15) via an axially flexible, resilient element (60) such that a headpiece (36) of the nonreturn valve is biased into the closing position.

**Revendications**

**1.** Dispositif de cathéter comportant

un cathéter à demeure (10) qui comprend un tube de canule (14), à l'extrémité arrière duquel est prévu un élément de raccord (15), et un orifice d'injection (17) débouchement latéralement,

et un mandrin (12), insérable dans le cathéter à demeure (10) à travers l'élément de raccord (15), qui s'étend dans l'état introduit jusqu'à l'extrémité avant de tube de canule, du côté du patient, et qui bloque alors l'extrémité avant su tube de canule en s'opposant à l'entrée de sang, un canal pour liquide (34) de tracé longitudinal étant ménagé entre le mandrin (12) et le tube de canule (14) et le mandrin (12) constituant, avec le tube de canule (14), une soupape à l'extrémité avant du canal de liquide (34),

**caractérisé en ce que**

la soupape est un clapet anti-retour (35) qui s'ouvre sous l'effet de la surpression dans le canal pour liquide (34) et se compose d'un élément de tête (35), situé à l'extrémité avant de mandrin, bloquant le canal pour liquide (34), et en ce que le mandrin est extensible en direction longitudinale de telle manière que l'élément de tête (36) s'écarte du tube de canule (14) en cas de surpression dans le canal pour liquide (34).

**2.** Dispositif de cathéter comportant :

un cathéter à demeure (10) qui comprend un tube de canule (14), à l'extrémité arrière duquel est prévu un élément de raccord (15), et un orifice d'injection (17) débouchant latéralement,

et un mandrin (12), insérable dans le cathéter à demeure (10) à travers l'élément de raccord (15), qui s'étend dans l'état introduit jusqu'à l'extrémité avant de tube de canule, du côté du patient, et qui bloque alors l'extrémité avant du tube de canule en s'opposant à l'entrée de sang, un canal pour liquide (34) de tracé longitudinal étant ménagé entre le mandrin (12) et le tube de canule (14) et le mandrin (12) constituant, avec le tube de canule (14), une soupape à l'extrémité avant du canal de liquide (34),

**caractérisé en ce que,**

le clapet anti-retour (35) se compose d'une zone de rétrécissement (38) susceptible de s'élargir élastiquement qui est située à l'extrémité avant du tube de canule (14) et enferme l'extrémité avant du mandrin (12).

**3.** Dispositif de cathéter comportant ;

un cathéter à demeure (10) qui comprend un tube de canule (14), à l'extrémité arrière duquel est prévu un élément de raccord (15), et un orifice d'injection (17) débouchant latéralement,

et un mandrin (12), insérable dans le cathéter à demeure (10) à travers l'élément de raccord (15), qui s'étend dans l'état introduit jusqu'à l'extrémité avant de tube de canule, de côté du patient, et qui bloque alors l'extrémité avant du tube de canule en s'opposant à l'entrée de sang, un canal pour liquide (34) de tracé longitudinal étant ménagé et le mandrin (12) constituant, avec le tube de canule (14), une soupape à l'extrémité avant du canal de liquide (34),

**caractérisé en ce que,**

le mandrin (12) est creux et contient le canal pour liquide (40) de tracé longitudinal et en ce que la soupape l'extrémité avant du mandrin (12) est réalisée sous forme d'un clapet anti-retour fendu (35), segments s'écartant radialement, qui s'ouvre sous l'effet de la surpression dans le canal pour liquide (40).

4. Dispositif de cathéter selon la revendication 2, caractérisé en ce que le clapet anti-retour (35) se compose d'un élément de tête (36) situé à l'extrémité avant du mandrin (12), de section transversale accrue, bloquant le canal pour liquide (34), et d'une partie d'extrémité (37), située à l'extrémité avant du tube de canule (14), enferment la partie de tête (36) et susceptible de s'élargir élastiquement.

5. Dispositif de cathéter selon l'une des revendications 1 ou 2, caractérisé en ce que la section transversale du mandrin (12), dans la zone du canal pour liquide (34), est ronde et que son diamètre est plus petit que le diamètre intérieur du tube de canule (14) afin de former le canal pour liquide (34).

6. Dispositif de cathéter selon l'une des revendications 1 ou 2, caractérisé en ce que le canal pour liquide (34) se compose de rainures de tracé longitudinal ménagées dans le mandrin (12).

7. Dispositif de cathéter selon la revendication 1, caractérisé en ce que le mandrin (12) comporte une spirale (39) dont les spires sont disposées côte-à-côte de façon lâche et constituent un canal pour liquide de forme spirale de façon que, en cas de surpression dans le canal pour liquide, la spirale s'étend axialement en augmentant la section transversale du canal de liquide.

8. Dispositif de cathéter selon la revendication 1, caractérisé en ce que le mandrin (12) est relié à l'élément de raccord (15) au moyen d'un élément axial (60) de sollicitation élastique (60) d'une manière telle qu'une partie de tête (36) du clapet anti-retour est sollicitée dans la position de fermeture.

**FIG.1**

**FIG.2**

**FIG.3**

FIG.4

FIG.5

FIG.6

FIG.7

FIG.8

FIG.9

FIG.10

FIG.11